Europäisches Patentamt

European Patent Office

Office européen des brevets

(11) Publication number: 0 546 634 A2

## EUROPEAN PATENT APPLICATION

(21) Application number: 92203827.8

(22) Date of filing: 09.12.92

(51) Int. Cl.5: **C12P 21/08**, C12N 5/10, C07K 15/06

(30) Priority: **13.12.91 US 807300**

(43) Date of publication of application:
**16.06.93 Bulletin 93/24**

(84) Designated Contracting States:
**AT BE CH DE DK ES FR GB GR IE IT LI LU MC NL PT SE**

(71) Applicant: **AKZO N.V.**
**Velperweg 76**
**NL-6824 BM Arnhem(NL)**

(72) Inventor: **Haspel, Martin Victor**
**14029 Berryville RD**
**Seneca, MD 20874-3519(US)**
Inventor: **Kobrin, Barry Jay**
**710 Horton Drive**
**Silver Spring, MD 20902(US)**
Inventor: **Crichton, Virginia Zewe**
**14829 Bauer Drive**
**Rockville, MD 20853(US)**

(74) Representative: **Hermans, Franciscus G.M. et al**
**P.O. Box 20**
**NL-5340 BH Oss (NL)**

(54) **Tumor associated monoclonal antibody 88BV59.**

(57) This invention relates to transformed B-cell lines derived from peripheral blood B-cells of cancer patients actively immunized with autologous tumor antigen and the monoclonal antibodies they produce. These monoclonal antibodies can be used in both diagnostic procedures and therapy for human cancers.

EP 0 546 634 A2

## DESCRIPTION OF THE INVENTION

This invention relates to a monoclonal antibody produced by a transformed B-cell line derived from B-cells of cancer patients actively immunized with autologous tumor antigen. This monoclonal antibody can be used in both diagnostic procedures and therapy for human cancers. Also part of this invention is the isolation and sequencing of the heavy chain and light chain variable regions and the identification of complementarity determining regions contained therein.

## BACKGROUND OF THE INVENTION

This invention relates to new human monoclonal antibodies that react specifically with antigens associated with particular cancers and to the production of transformed B-cell lines derived from peripheral blood B-cells of actively immunized patients. This invention also relates to diagnostic procedures and cancer therapy using these monoclonal antibodies.

Currently available treatments for cancer, particularly radiation therapy and chemotherapy, are based upon the rationale that cancer cells are relatively more sensitive to these treatments than normal cells. However, severe toxicity for normal tissues imposes major limitations to these therapies. In contrast, antibody molecules exhibit exquisite specificity for their antigens. Researchers have therefore sought to isolate antibodies specific for cancer cells as the "long-sought 'magic bullet' for cancer therapy" (Science, 1982, 216:283).

Antibodies are protein molecules normally synthesized by the B-cell lymphocytes produced by bone marrow and carried in the blood stream. For any antigen entering the body, i.e., any foreign molecule from a simple organic chemical to a complex protein, antibodies are produced which recognize and attach to that particular chemical structure. The unique chemical structure on the antigen to which a particular antibody can bind is referred to as an antigenic determinant or epitope. B-cell lymphocytes in the body, referred to as B-cells, lymphocytes, or leukocytes, exist as hundreds of millions of different genetically programmed cells, each producing an antibody specific for a different determinant. An antigen, which stimulates antibody production, can have several determinants on its surface. On encountering an antigen, a B-cell carrying on its surface an antibody specific for a determinant on that antigen will replicate. This clonal expansion results in many daughter cells that secrete that antibody into the blood stream.

Because of the specificity of antibodies in recognizing and binding to antigens, it was desired to produce antibodies in quantity that are specific for a single determinant, thus binding only to antigens or tissues having that particular determinant.

B-cells do not grow in a continuous culture unless they have been altered by hybridization with an "immortal" cell or by being transformed with either viral or tumor DNA. Monoclonal antibodies are produced by B-lymphocyte cell lines that have been transformed, either spontaneously or intentionally, with a lymphotropic virus such as Epstein-Barr Virus (EBV). Transformation can also be accomplished using other transforming agents, such as viral DNA and cellular DNA. These cells, unlike hybridoma cells, possess a normal human diploid number (46) of chromosomes.

Monoclonal antibodies are synthesized in pure form uncontaminated by other immunoglobulins. With monoclonal antibody producing cells it is possible to produce virtually unlimited quantities of an antibody that is specific for one determinant on a particular antigen.

It has been believed that if antibodies specific for particular cancer cells were available, they could be used in various methods of treatment and diagnosis. Such antibodies could inactivate or kill particular tumor cells merely by attaching to the cell at the determinant for which they are specific. Alternatively, these antibodies may bind to the surface of effector lymphocytes or macrophages, converting them into tumor antigen-specific killer cells.

Monoclonal antibodies can also increase the specificity of chemotherapeutic drugs, toxins and radioactive isotopes, thus increasing their efficacy while decreasing their toxicity by being conjugated to them. In addition, antibodies conjugated with radionuclides or metallic tracers can be used for proton emission (PET) and nuclear magnetic resonance (NMR) imaging for in vivo diagnosis and localization of metastases. The antibodies can also be used for detecting the presence of tumor antigens in blood, as a diagnostic and/or prognostic test for cancer. Also, monoclonal antibodies can be used to isolate tumor antigens for potential use in a standardized vaccine.

In addition to the constant region, which is characteristic of the species, antibodies contain variable regions on both the heavy chain ($V_H$) and the light chain ($V_L$). These variable regions are the parts of the antibody that determine binding specificity and the variable region on every antibody that binds to a particular epitope is different from the variable regions on antibodies that bind to different epitopes. For the purpose of using antibody specificity to target drugs and radiometals for therapy and imaging, we believe that advantages can possibly be obtained by using only the portions of the antibodies active in binding for preparing immunoconjugates. Identifying and sequencing the complementarity determining regions of the heavy and light chains also provide information for synthesizing chimeric and multifunctional antibodies. For this reason, we isolated and sequenced the variable regions and determined the regions in the sequence that function to bind epitopes.

DESCRIPTION OF THE PRIOR ART

Past attempts at deriving monoclonal antibodies specific for human cancers have taken two routes with respect to B -cells: 1) B-cells have been extracted from spleens of mice that were immunized against human tumors, U.S. Patent 4,172,124; and 2) human B-cells have been extracted from either peripheral blood or from lymph nodes draining tumors in cancer patients. Neither approach has yielded satisfactory results.

Mice immunized against human tumors have too broad a reactivity. That is, most of the mouse monoclonal antibodies generated react with human antigens present on normal as well as on tumor tissue. An antibody that reacts only with tumor cells is very difficult to select from among the large variety of antibodies produced. For example, 20,000 hybridomas derived from mice immunized with human small-cell lung carcinoma were screened for reactivity with tumor cells (Science, 1982, 216:283). In contrast to a very low frequency (<0.4%) observed by this research group, obtaining activated B-cells by the method used in the present invention results in up to 16% of the immortalized B-cells derived from immunized colon patients producing monoclonal antibodies that react specifically with tumor cells. In addition, monoclonal antibodies derived from mouse B-cells have limited potential for application in cancer therapy. After repeated administration they stimulate the human immune system to produce "anti-mouse" antibodies which, in clinical trials, have been shown to significantly diminish the efficacy of mouse monoclonal antibodies. The use of our human monoclonal antibodies can circumvent these difficulties. Use of the variable regions alone, or just the complementarity determining regions (CDR's), provides even less likelihood of immunogenicity as well as other advantages. The ability of smaller molecules, such as $V_H$ or even a single CDR, to rapidly clear from the body may be utilized in designing rapid in vivo diagnostic agents.

Another apparent difference between human and mouse monoclonal antibodies is their immunohistochemical staining pattern. Previous studies with mouse antibodies have demonstrated that there is often a heterogenous staining of cells within tumor sections. This pattern of reactivity has been attributed by some authors to antigenic heterogeneity of tumor cells (Hand et al., Cancer Research, 43:728-735, 1983). In contrast, the human monoclonal antibodies developed by our strategy were homogeneous in terms of their reactivity with tumors to which they did react. A plausible explanation for the heterogenous staining of mouse monoclonal antibodies is that it is a reflection of the murine immune recognition of phase- or cell-cycle-specific differentiation antigens abundant on the tumor cells rather than putative tumor associated antigens. It is not unreasonable to expect that when one immunizes mice with human tumor cells there would be substantial antigenic competition resulting in the more abundant and more predominant tissue-type and differentiation antigens successfully competing with relatively minor tumor associated antigens for immune responsiveness by the host. Thus, autologous immunization of man may result in the elicitation of antibodies against the group of antigens normally poorly immunogenic in mice. This evidence suggests that humans and mice may respond to different tumor antigens. In concert with this hypothesis is our finding that none of the first 36 human monoclonal antibodies we produced appeared to react with carcinoembryonic antigen (CEA), an antigen frequently recognized by murine monoclonal antibodies made against human tumor cells.

The majority of past attempts to develop human monoclonal antibodies have used B-cells extracted from either peripheral blood or lymph nodes from patients bearing tumors. It was believed that the presence of the antigenic tumor would cause a tumor-bearing individual to mount an immune response against his tumor and produce specifically immune B-cells. Thus, B-cells were taken from lymph nodes draining tumors in cancer patients or from circulating lymphocytes found in peripheral blood. However, prior to the present invention, there has been limited success in creating tumor-specific monoclonal antibodies.

The major problem in creating monoclonal antibodies specific for human tumor antigens has been the inability to find a source of specifically immune B-cells (Science, 1982, 216:285). In humans, the initial foci of cancer cells tend to grow over long periods of time, from 1% to 10% of the human lifespan, before there is any palpable clinical evidence of the disease. By this time patients are immunologically hyporesponsive to their tumors, or possibly immunologically tolerant. Thus, prior to the present invention, human monoclonal antibodies reactive with tumor cells could not reproducibly be obtained.

We have developed a new and more effective approach for obtaining monoclonal antibodies by using peripheral blood B-cells from patients immunized with cells from their own tumors in specific vaccine preparations. To achieve active specific immunotherapy, patients were immunized with autochthonous tumor cells, that is, cells from their own tumors. This approach was taken based on our theory that tumor cells express tumor-specific antigens.

Humans mounting an objective immune response against tumor cells were specifically found to be a good source of activated B-cells. We have shown that the peripheral blood of patients who had been actively immunized against their own tumors is an abundant source of such activated B-cells.

We demonstrated in clinical studies that an objective immune response is generated on treating patients having the particular cancer by skin testing, i.e., delayed cutaneous hypersensitivity (DCH). Immunized patients showed delayed cutaneous hypersensitivity to their own colorectal cancers. In addition, the monoclonal antibodies developed from the immunized patient's B-cells reacted with tumors of the same histological type in other patients. These results indicate that the patient's humoral immune response, production of antibodies, is directed against colorectal cancer generally and is not unique to the immunized patient's own tumor. This general response is especially important for the development of a standardized vaccine.

The generation of B-cells that produce antibodies having reactivity specific for epitopes on tumor cell associated antigens, particularly cell surface antigens as in the majority of cases, is an advantageous result that was speculative, at best, when the immunization studies were begun. Only the therapeutic effects of immunization were observed and measured during the animal studies on which the human immunization procedures were based, not the production of tumor specific antibodies.

The general immune response accompanied by an improvement in the subject's condition was indicative of a cellular response in which macrophages and T-cells become activated in the presence of tumor cell antigens and destroy the tumor cells. Although an antibody response would predictably be triggered by immunization under most circumstances, the time course of the antibody response and the cellular response would in most instances be different. Moreover, the fact that the patients were being immunized with autologous tumor cells, i.e., the patient's own tumor cells, and the experience of previous investigators that little or no antibody production is triggered by a patient's own tumor, made our discovery that B-cells that produce tumor specific antibodies are generated after immunization an unexpected beneficial result.

This invention is the result of our discovering ways to prepare successful vaccines for active specific immunization; developing procedures for extracting immunized human B-cells; and developing methods for sustained and continuous production of human monoclonal antibody producing cell lines and the production of monoclonal antibodies.

## SUMMARY OF THE INVENTION

One object of the present invention was to develop human monoclonal antibodies specifically reactive with tumor-associated antigens and minimally reactive with antigens presented on non-tumor tissue. Such antibodies provide a means for detecting and diagnosing tumors and for treating patients with particular types of cancer. A further object was to identify a human monoclonal antibody that exhibits these properties, and to isolate, immortalize and culture the cell line producing this antibody. We also had as our objective sequencing the variable regions of the selected antibody, and isolating and identifying the portion of the heavy and light chains that actually bind to epitopes on these antigens, the complementarity determining regions.

This invention comprises the EBV transformed human B-cell line CO88BV59, which produces human IgG$_3$ $x$, specifically reactive with an epitope found on a human colon tumor antigen, as well as identifying and sequencing the complementarity determining regions of this antibody.

## DETAILED DESCRIPTION OF THE INVENTION

This invention is, specifically, a human diploid cell line, an immortalized human B-cell line that we transformed by exposure to EBV, designated CO88BV59. It produces a human IgG$_3$ $x$ antibody specifically reactive with an epitope on a colon tumor antigen defined in copending application USSN 07/343,475, filed February 28, 1989. This antigen was first identified using human IgM antibody 16-88 defined in US. Patent 4,997,762, which issued from our copending application USSN 07/038,811 filed April 15, 1987, included herein in its entirety be reference. IgG$_3$ $x$ 88BV59 and IgM 16-88 recognize the same tumor associated antigen, but react with different epitopes on that antigen.

Example I: Preparation of Sensitized B-Cells

A. Patient Selection.

Patients undergoing surgical resection of colon or rectal cancers were selected for a randomized trial of active specific immunotherapy. Randomization was done with stratification according to pathologic stage and tumor was obtained from all patients who met the clinical criteria. Candidates for the study were colorectal cancer patients with no previous history of cancer, who had received no prior chemotherapy or radiation therapy, and who were in suitable medical condition to comply with the outpatient treatment protocol. Patients eligible for the trial were those with tumor extending through the bowel wall (Astler-Coller B2), positive lymph nodes (stages C1, C2) or patients with metastatic disease (stage D). Within these classifications, patients were randomly selected for participation in treatment and non-treatment groups. Randomization cards were computer generated and sequentially drawn from each category postoperatively.

B. Tumor Acquisition.

After surgical resection the bowel specimen was taken immediately to the hospital pathology department and opened under sterile conditions. Tumor tissue was excised, placed in sterile tubes containing Hank's Balanced Salt Solution (HBSS) containing 50 $\mu$g gentamicin per ml and carried immediately on ice to the laboratory for processing and freezing.

C. Dissociation of Solid Tumor and Colon Mucosa.

The tissue dissociation procedure of Peters et al (Cancer Research, 39:1353-1360, 1979) was employed using sterile techniques throughout under a laminar flow hood. Tumor tissue was rinsed three times in the centrifuge tube with HBSS and gentamicin and transferred to a petri dish on ice. Scalpel dissection removed extraneous tissue and the tumor was minced into pieces approximately 2 to 3 mm in diameter. Tissue fragments were placed in a 75 ml flask with 20-40 ml of 0.14% (200 units/ml) Collagenase Type 1 (Sigma C - 0130) and 0.1% (500 Kunitz units/ml) deoxyribonuclease type 1 (Sigma D - 0876) (DNAase 1, Sigma D-0876) prewarmed to 37°C. Flasks were placed in a 37°C waterbath with submersible magnetic stirrers at a speed which caused tumbling, but not foaming. After a 30-minute incubation free cells were decanted through three layers of sterile medium-wet nylon mesh (166t: Martin Supply Co., Baltimore, Maryland) into a 50 ml centrifuge tube. The cells were centrifuged at 1200 rpm (250 x g) in a refrigerated centrifuge for 10 minutes. The supernatant was poured off and the cells were resuspended in 5-10 ml of DNAase (0.1% in HBSS) and held at 37°C for 5-10 minutes. The tube was filled with HBSS, washed by centrifugation, resuspended to 15 ml in HBSS and held on ice. The procedure was repeated until sufficient cells were obtained, usually three times for tumor cells. Cells from the different digests were then pooled, counted, and cell viability assessed by the trypan blue exclusion test. The cells were centrifuged for a final wash prior to cryopreservation.

D. Cryonreservation.

Optimal cryopreservation was a primary concern. For vaccine preparation, the dissociated tumor cells were adjusted to 5-8 x $10^7$/ml in HBSS and added in equal volume to chilled 2 X freezing medium containing 15% dimethylsulfoxide (DMSO) and 4% human serum albumin (HSA). The final suspension of 2 to 4 x $10^7$ cells/ml were placed in 1.2 ml Nunc freezer vials. For DCH cell testing the procedure was the same except that no HSA was used. In both cases, in preparation for freezing, the Nunc vials were transferred on ice to a Cryo-Med model 990 Biological Freezer with a model 700 Controller and a model 500 Temperature Recorder for controlled-rate freezing. Care was taken that the temperature of the individual vials, including the monitor vial, was uniform at the beginning of the freezing process. Vials were cooled at a controlled rate of - 1°C/min to a final temperature of -80°C. The vials were transferred in liquid nitrogen to liquid nitrogen storage.

E. Clinical Protocol.

Patients with tumors of the appropriate pathologic stages were randomized to receive either the autologous tumor cell-BCG vaccine or to have no further therapy. The stage D patients all received 5-fluorouracil chemotherapy and all patients with lesions below the peritoneal reflection (rectal cancer) received 5040 rads of pelvic X-irradiation two weeks after immunotherapy was completed. The vaccines were started at 4-5 weeks after tumor resection to allow sufficient time for recovery of immunologic suppression induced by anesthesia and surgery. At 3-4 weeks after resection both control and treatment patients were skin tested with standard recall antigens as well as graded doses of their autologous tumor cells. Recall antigens used were: Mumps skin test antigen, USP, Eli Lilly, Indianapolis, Indiana; Aplisol, PPD, (Tuberculin Purified Protein Derivative), Parke-Davis, Detroit, Michigan; Trichophyton, diluted 1:30, Center Laboratories, Port Washington, New York; and Candida albicans diluted 1:100, Center Laboratories, Port Washington, New York, 0.1 ml of each was placed intradermally on the forearm and examined for erythema and induration at 24 and 48 hours.

Patients selected for treatment protocol received 3 weekly intradermal vaccine injections consisting of $10^7$ irradiated, autologous tumor cells and $10^7$ BCG in the first 2 vaccines with $10^7$ tumor cells alone in the final. Fresh-frozen Tice BCG, supplied by Dr. Ray Crispen, University of Illinois Medical Center, Chicago, Illinois, was stored at -70°C. The first vaccine was placed on the left anterior thigh approximately 10 cm below the groin crease, the second in a comparable location on the right thigh and the third in the right deltoid area.

F. Preparation of Vaccine.

The criteria for successful vaccines are listed in Table 1. On the day of the first and second vaccinations, the vial was rapidly thawed in a 37°C waterbath, tumor cells were diluted slowly to 15 ml in HBSS, washed once by centrifugation at 1200 rpm and resuspended to 15 ml in HBSS. Cell counts and viability determinations were made using the trypan blue exclusion test. Viability ranged between 70 and 90%, with a mean of 80%. The cells were washed once by centrifugation at 1200 rpm and resuspended to 15 ml in HBSS. The suspension of tumor cells was placed on ice and irradiated at 4020 rads/min for a total of 20,000 rads. The volume of the cell suspension was adjusted such that $10^7$ viable tumor cells remained in the tube (1.3 x $10^7$ viable cells are included to allow for cell loss in tubes and syringes, and for the possibility of approximately 20% misidentification of lymphoid cells). The cells were centrifuged, the supernatant removed and $10^7$ BCG were added in a volume of 0.1 ml. HBSS was added in sufficient quantity for a final volume of 0.2 ml. The third vaccine was similarly prepared, omitting the BCG.

The vaccine suspension was drawn up through a 20 gauge needle into a 1.0 ml tuberculin syringe. The 20 gauge needle was replaced with a 27 gauge needle for the intradermal injection, and the syringe was placed on ice for transport to the clinic.

The patients were observed closely after each vaccine for erythema and induration at the site of injections, fever, lymphadenopathy or any adverse reactions. The first two vaccine sites ulcerated after 2-3 weeks but always healed within 10 to 12 weeks.

Example II: Production of Cells Producing Human Monoclonal Antibodies

A. Removal and Processing of Immunized B-Cells from Patients.

Patients were bled at the time of the second immunization, one week after the first immunization, and at the time of the third vaccination, one week after the second immunization. Venous blood was collected aseptically in the presence of preservative-free heparin (O'Neill, Jones and Feldman, St. Louis, Missouri) at a final concentration of 17 units/ml. The blood was maintained at room temperature and transported to the laboratory expeditiously, within a few hours of collection.

The blood, diluted 1:2 with calcium and magnesium-free HBSS, was layered (4 ml) over 3 ml of lymphocyte separation medium (LSM, Litton Bionetics) and centrifuged in a 15 ml centrifuge tube for 30 minutes at 400 x g. The cells at the interface were removed, diluted with three times their volume of HBSS and pelleted (1000 rpm for 10 minutes). The peripheral blood lymphocytes were resuspended in 10 ml of serum-free Hepes-buffered Dulbecco's MEM (DMEM), counted and viability determined.

6

## B. EBV Transformation Procedure.

Peripheral blood B-cells from immunized patients were intentionally exposed to transforming agents, resulting in continuously growing cell lines that produce monoclonal antibodies. We have used EBV as the transforming agent, although any effective lymphotropic virus or other transforming agent able to transform the B-cells to grow in continuous culture and still produce monoclonal antibodies specific for tumor associated antigens can be used.

By our method, heparinized blood was separated on an LSM gradient and the mononuclear cell fraction was collected at the interface. The mononuclear cell fraction can either be used at this point or cryopreserved for future transformation.

The lymphocytes, either fresh or cryopreserved, either unfractionated or depleted of some non-B cells, were counted and between 2 and 6 x $10^6$ cells were pelleted. The pelleted cells were resuspended in 5 ml of freshly harvested Epstein Barr Virus in the form of undiluted B95-8 supernatant fluid harvested from a 4 - 6 day old culture of B95-8 cells, clarified by centrifugation at 2,000 rpm for 15 minutes at 4°C and filtered through a 0.8 micron filter to insure that all cells had been removed. The B95-8 cell line was obtained from Dr. G. Tostado, Division of Biologics, FDA. The cells and EBV were incubated at 37°C for 90 minutes for virus adsorption. During virus adsorption, the cells were agitated periodically.

The cells were plated into wells containing irradiated feeder cells (such as J774). The mouse macrophage line J774 (ATCC, Rockville, Md.) was irradiated (20,000 rads) and then cryopreserved. For this method feeder cells were thawed and then plated (5 x $10^3$ cells/well) into 96 well plates one day before the EBV transformation of the B-cell line.

The cell culture medium was changed twice per week for up to 6-8 weeks. Screening of supernatant fluid from wells exhibiting extensive cell growth to select those synthesizing human immunoglobulin and the culturing of selected cell lines was performed according to the procedures described in U.S. Patent 4,828,911 for selection and culturing of monoclonal antibody producing cells.

Cells selected for producing human immunoglobulin were cultured and selected for tumor reactivity by screening against human tumor xenografts. 88BV59 was screened against a block consisting of four frozen sections of human colon carcinoma xenografts from nude mice. Xenograft sections and direct labeling of antibody were used in order to avoid the presence of human immunoglobulin.

## C. Production of Monoclonal Antibodies.

Human monoclonal antibody producing cells were grown in RPMI 1640 medium (Gibco, Grand Island, New York) supplemented with 10% fetal bovine serum, 3 Mm L-glutamine and 5 $\mu$g/ml gentamicin. The medium was in some cases further supplemented with 25% D-glucose (final concentration 0.25%). The cells were at 37°C (35-38°C) under a humidified atmosphere of 7.5% $CO_2$ in air. The antibody was harvested from the highly metabolized spent medium by pelletizing the medium free of cells (e.g., by centrifuging at 500 rpm for 15 minutes).

## Example III: Reactivity of Monoclonal Antibodies to Normal and Tumor Tissue

Most of the antibodies exhibited substantially reduced binding to normal colonic mucosa. The antibodies reactive with paraffin sections were also tested for reactivity with normal tissue. 88BV59 showed negative reactivity with the following normal human tissues: ovary, uterus, testes, vagina, adrenal glands, prostate, thyroid, thymus, lymph nodes, spleen, bone marrow, myocardium, cerebral cortical cells, skin, muscle and hemopoietic cells. 88BV59 exhibited slight reactivity with the following tissues: colon (brush border and superficial glands), small intestine (brush border and superficial glands), stomach (gastric pits and superficial glands), esophagus (glands), pancreas (some ductal and exocrine glandular epithelium), kidney (50% of collecting tubules), cervix (epithelial lining (2/3 tissues were positive)), breast (acini and ductal epithelium), lung (some alveolar and bronchial cells), brain (astrocytes (2/3 tissues were positive)), spinal cord (neuropil), skin (50% of glands in dermis) and liver (bile ducts). Reactivity of 88 BV59 with human tumor cell lines is shown in Table 2. Table 3 shows the reactivity of 88BV59 with tumor tissue specimens.

In addition to providing monoclonal antibodies reactive with tumor cell surface antigens for the in vivo diagnosis and immunotherapy of cancer, the invention provides monoclonal antibodies that will be useful as probes to isolate and characterize the antigens relevant to human cancer immunity. These antigens may ultimately prove useful as a tumor vaccine. In addition, the generation of antibody producing diploid cells adds a dimension of genetic stability to the production of human monoclonal antibodies reactive with tumor cell surface antigens.

The foregoing describes the formation of novel monoclonal antibodies specific for certain tumors, monoclonal antibody producing cell lines, and methods for their preparation. It will be understood that many variations and modifications of the techniques disclosed herein are available to those of ordinary skill in the relevant art and that such variations and modifications are contemplated as being within the scope of the invention.

This invention is specifically directed to the cell line producing the $IgG_3$ x human monoclonal antibody 88BV59, which was deposited with the American Type Culture Collection, 12301 Parklawn Drive, Rockville, Maryland 20852, USA, on December 13th, 1990. 88BV59 was identified using the antigen reactive with human IgM 16-88, produced by diploid cell line LiCo 16-88, taught in U.S. Patent 4, 997,762 issued from U.S. Patent Application 038,811, filed April 15, 1987, a divisional of USSN 697,078, filed January 31, 1985. The cell line deposited is identified as follows:

| Identification | Accession Number |
|---|---|
| Human B-Cell Derived Cell Line, CO88BV59-1 | CRL 10624 |

EXAMPLE IV: Cloning and Sequencing 88BV59

Total RNA from 88BV59 (diploid) cells was prepared by the method of Chomczynski and Sacchi (Analytical Biochemistry, Vol. 162:156-159, 1987). Briefly, cells were pelleted, imploded in 4 Molar guanidium isothiocyanate, 25 mM Sodium citrate pH 7.0, 0.5% Sarcosyl and 0.1 M 2-Mercaptoethanol and RNA was extracted after the addition of 0.02 M Sodium acetate pH 4.0, 1 volume of phenol and 0.2 volume chloroform. Samples were centrifuged 10,000 XG for 20 minutes at 4°C and the aqueous phase transferred to a fresh tube and re-precipitated with one volume of isopropanol for one hour at -20°C. After a resuspension in the guanidium isothiocyanate-based solution and reprecipitation with isopropanol cell pellets were washed with 75% ethanol and resuspended in 0.5% SDS. Oligo dT cellulose chromatography (Aviv and Leder PNAS Volume 69:1408-1412, 1972) was performed to select for poly A (+) mRNA according to standard procedures.

A. cDNA Synthesis.

1.2 μgs of 88BV59 poly A (+) was combined with 1 μg of Promega NotI oligo dT primer adapter (5' CAATTCGCGGCCGC(T)₁₅3') by heating at 70°C and chilling quickly on ice. (500 μM)f of dNTPs and 200 units of Life Technologies Inc.'s Moloney Murine Leukemia Virus RNase H' reverse transcriptase (Superscript)™ were added and incubated in 50 mM Tris HCl pH 8.3, 75 mM KCl 3 mM MgCl₂ and 10 mM dTT for 60 minutes at 37°C. Second strand synthesis was performed via the addition of 187 μM dNTPs, 40 units E. coli DNA Polymerase I,15 units E. coli DNA Ligase and 1.4 units E. coli RNase H in 19 mM Tris HCl pH 6.9, 90 mM KCl 4 mMgCl₂ 125 μM ß-Nad and 50 mM ammonium sulfate. After incubation at 16°C for two hours the reaction was stopped with 50 mM EDTA and the cDNA was purified through phenol extraction and ethanol precipitation. The 5' termini of the double stranded cDNA were polished by the addition of 10 units of T₄ DNA Polymerase in the presence of 225 μM dNTPS in 0.3 X of the second strand cDNA synthesis buffer. After incubation at 37°C for ten minutes the reaction was terminated with 20mM EDTA 0.2% SDS phenol extracted and ethanol precipitated. The resulting polished blunt ended double stranded cDNA was resuspended in H₂O and used further.

Promega's Ribaclone™ ECoRI linker ligation system I was used to methylate any internal ECoRI sites in the double stranded cDNA and subsequently to add ECoRI sites to the blunt ended ds cDNA termini. To the cDNA 10 units of ECoRI methylase and (100 μM)f S-adenosyl-L-methionine were added in 100 mM Tris HCl pH 8.0, 10 mM EDTA and incubated at 37°C for 15 minutes. After heat inactivation at 70°C for ten minutes and phenol extraction the methylated cDNA was ethanol precipitated. To one-half of the methylated cDNA ECoRI linkers were added, in 30 mM Tris HCl pH 7.8, 10 mMgCl₂, 10 mM ATP, 1 mM DTT, 100 μg/ml BSA, 25 Weiss units of T4 DNA Ligase catalyzed the addition of ECoRI linkers, present in a 50-fold M excess, to the cDNA termini by incubation overnight at 15°C. Following heat inactivation of the ligation reaction at 70°C for ten minutes, the cDNA was digested with 20 units each of restriction enzymes ECoRI and NotI at 37°C for 2 hours. EDTA and SDS were added to (20 mM)f and (0.2%)f respectively, and following phenol extraction the aqueous phase was passed over Sepharacryl® S-400 spin columns to remove excess linkers and very small cDNAs. After addition of (2 M)f ammonium acetate and 2 volumes of ethanol the cDNA was precipitated and resuspended in 10 $\mu$l dH$_2$O.

B. Library Construction.

5 $\mu$L of 88BV59 cDNA was ligated to 1 $\mu$g of Promega Corporation's $\lambda$gt11 Not1-ECoRI arms and the resulting DNA was packaged in vitro into phage particles using Gigapak Gold™ (Stratagene Corporation, La Jolla, CA) according to the manufacturer's instructions. The phage packaged DNA was plated on 150 mm agar plates at a density of approximately 50,000 per plate.

A human IgG$_3$ heavy chain constant region specific probe was derived directly from the unligated 88BV59 cDNA. An oligonucleotide complementary to amino acids 115-121 of the IgG$_3$ heavy chain CH$_1$ region, i.e., 5' TCCACCAAGGGCCCATCGGTC3', extending in a 3' direction and another oligonucleotide complementary to amino acid 222-217, i.e., 5' TTTCTTGTCCACCTTGGTGTT3', extending in a 5' direction were synthesized on a Milligen synthesizer and purified according to manufacturers specifications. 100 pM of oligonucleotides were utilized in a standard polymerase chain reaction (PCR™) using Cetus' Ampli-Taq™ polymerase with 1 $\mu$L of 88BV59 cDNA with denaturation at 94°C for one minute and annealing at 52°C for one minute and extension at 72°C for one minute. The PCR generated a 291 base paired human IgG$_3$ CH$_1$ specific fragment. The fragment was $^{32}$P radiolabeled with the Boehringer Mannheim random priming kit (Boehringer Mannheim, Indianapolis, IN) according to manufacturer's protocol and 500,000 phage were screened with the $^{32}$P labeled IgG$_3$ fragment. Plaque hybridization was carried out in 6X SSC, 1X Denhardts reagent 0.01% SDS and 50 $\mu$g/mL denatured salmon sperm DNA at 65°C for 18 hours. Final washes were in 0.1X SSC, 0.1% SDS at 61°C.

An oligonucleotide complementary to positions 109-114 in the human C$x$ gene, i.e., 5' ACTGTGGCTGCACCATCT3', was synthesized and 5' end-labeled with T4 polynucleotide Kinase (New England Biolabs, Beverly, MA) and utilized to screen 150,000 plaques. Hybridization was identical to that of the heavy chain except that it was performed at 42°C for 16-18 hours. Final washes for the light chain probe were in 1X SSC 0.1% SDS at 42°C.

Positive clones were identified from both libraries after autoradiography and either seven (for the light chain) or eight (for the heavy chain) individual plaques were isolated after three rounds of plaque purification. PCR amplification utilizing Promega Corporation's $\lambda$gt11 forward and reverse primers annealed at 42°C was performed using an aliquot of a 1 mL lysate of each $\lambda$ clone. Insert sizes were determined by agarose gel electrophoresis.

C. Subcloning of Phage DNA.

Independent phage each with the expected size insert for full length IgG$_3$ or $x$ light chains were grown in 40 ml cultures at a multiplicity of infection of 100 cells to one phage at 37°C for 6 hours. Following removal of debris, supernatants were digested with 1 $\mu$g/ml of DNase and RNase at 37°C for 2 hours and phage were precipitated with 5% polyethylene glycol and 0.5M sodium chloride for 2 hours at 4°C. Phage were pelleted and following chloroform extraction which removed excess polyethylene glycol, phage DNA was extracted twice with an equal volume of phenol followed by a single chloroform extraction. Addition of 2 volumes of ethanol facilitated the spooling of the $\lambda$ DNA and its transfer and solubilization in 10 mM Tris HCl pH 7.4 1 mM EDTA. $\lambda$ DNAs were cleaved with ECoRI and NotI (20-40 units each) at 37°C for 2 hours. Ig cDNA inserts were purified from 0.7% low melt agarose gels according to the protocols of FMC Bioproducts, Rockland, ME. pGEM11$_{fz}$(-) (Promega Corporation, Madison, WI) vector was doubly cleaved with ECoRI and NotI and following enzyme heat inactivation the 5' terminal phosphates were removed by calf intestinal alkaline phosphatase (CIAP) (Boehringer Mannheim, Indianapolis, IN) by incubating 0.5 units of CIAP at 37°C for 30 minutes in 10 mM Tris HCl pH 8.0, 0.1 mM EDTA. Following ethanol precipitation the vector was ligated to gel purified insert at room temperature for 2-3 hours and the ligation was transformed into competent TG-1 cells (Amersham Corporation, Arlington Heights, IL) according to Maniatis et al. Clones containing the immunoglobulin heavy and light chain inserts were identified by a small scale plasmid growth and analysis of plasmid DNAs cleaved with ECoRI and NotI and resolved on agarose gels as described in Maniatis et al.

D. PCR-DERIVED SUBCLONING OF PHAGE DNA.

PCR amplification of independent phage inserts utilizing $\lambda$gt11 forward and reverse primers (Promega Corporation, Madison, WI) annealed at 42°C yielded phage with expected sizes of full length immunoglobulin heavy and light chain cDNAs. The PCR products were digested with ECoRI and NotI, purified

and ligated to pGEM11fz(-) ECoRI NotI cleaved vector. DNA from transformants with either Ig H or L inserts was prepared according to Maniantis et al. and sequenced using the dideoxy method with Sequenase™ - (US Biochemical Corporation, Cleveland, OH) according to the manufacturer's instructions. Promega Corporation's $T_7$ polymerase promotor-specific oligonucleotide located immediately 5' of the immunoglobulin inserts was used to sequence the 5' portion of both $V_H$ and $V_L$ cDNAs. An oligonucleotide complementary to amino acids 113-110 in the human $J_H$ (i.e., 5'TGAGGAGACGGGTGACC3') was used to further sequence the $V_H$ region. Subsequently, separate oligonucleotides complementary to amino acids +1 to +6 in both $V_H$ and $V_L$ were synthesized. For PCR subcloning a kappa light chain oligonucleotide complementary to the $C_x$ constant region amino acids 214-209 was used in a PCR reaction along with its appropriate $V_x$ amino acids 1-6 specific oligonucleotide. The modified Fab' heavy chain fragment comprising amino acids 1-241 was derived by PCR utilizing a oligonucleotide complementary to amino acids 241-236 and one complementary to amino acids 1-6 in $V_H$. Similarly for Fab construction a Fd heavy chain fragment was derived by PCR utilizing an oligonucleotide complementary to amino acids 222-216 and the aforementioned 5' $V_H$ amino acid 1-6 oligonucleotide. All our amino acid enumerations are according to the convention of Kabat et al. (Sequences of Proteins of Immunological Interest, Fourth Edition, NIH, 1987). The appropriate restriction enzyme sites were placed at the 5 and 3' end of each oligonucleotide to facilitate subcloning into a modified version of the bacterial expression vector pSWV$_H$POLY, a gift of Greg Winter of the MRC, Cambridge, England.

Individual subclones, two for the Fab and two for the Fab', were analyzed and sequenced using the dideoxy method with Sequenase™.

The Fab' cDNA in pSWV$_H$POLY had its 5' and 3' termini modified by the inclusion of appropriate restriction sites via PCR and utilizing the pSWV$_H$POLY Fab' cDNA the PCR was performed to transfer this entire coding portion of the cDNA to the pET system (Novagen Corporation, Madison, WI).

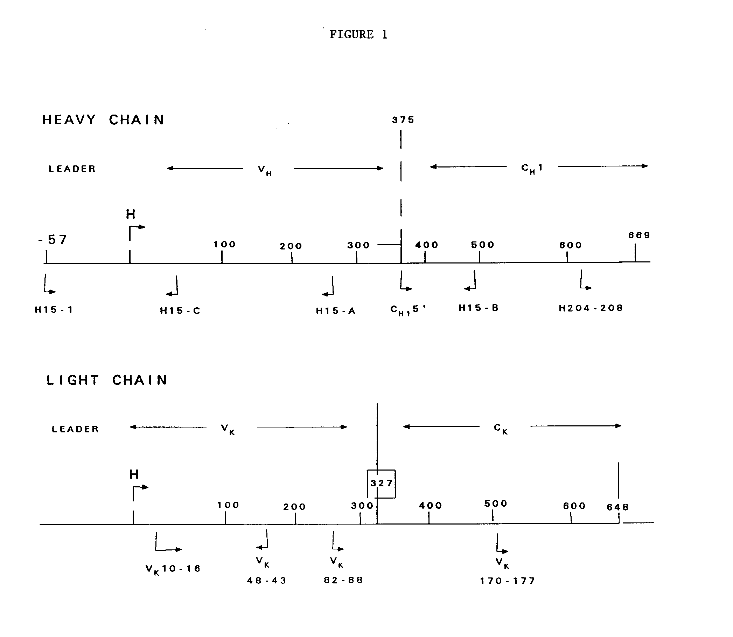

The complete light chain and Fab' heavy chain sequence was determined for the pET system Fab and the complete sequence of the Fab in pSWV$_H$POLY was determined independently for two separate subclones. A schematic describing the strategy utilized to sequence these clones is shown in Figure 1. Table 4 protrays the actual nucleotide sequence and location of the oligonucleotides used in deriving the sequence of 88BV59 heavy and light chains. The sequence of the complete heavy chain from the leader through amino acids 242 is shown in Figure 1. 88BV59 uses $V_H$III and a D region which may have resulted from intra D-D recombination and/or gene conversion along with somatic mutation. It is radically different from any germ line D region. It utilizes germ line $J_{H3}$.

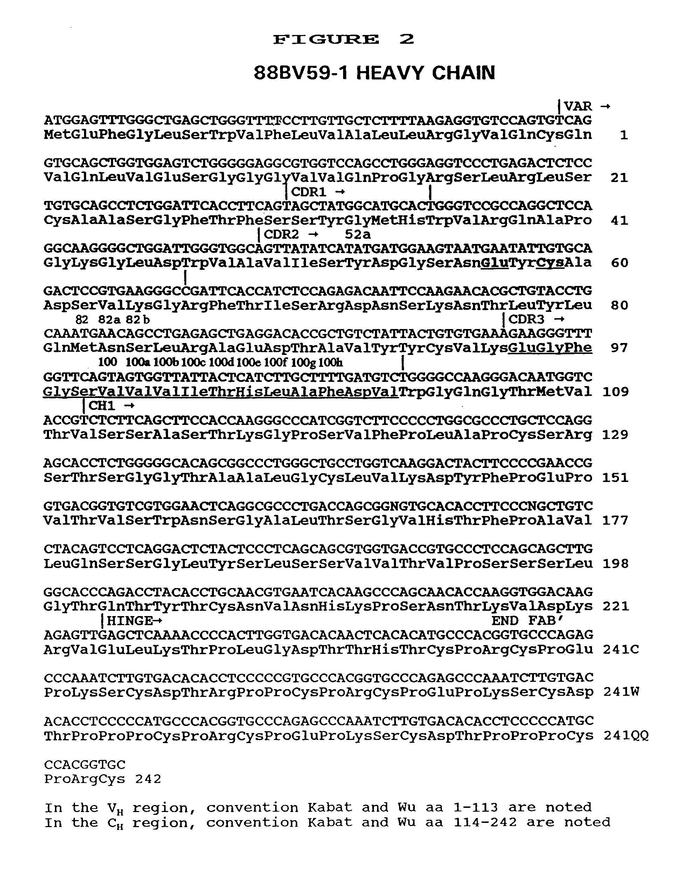

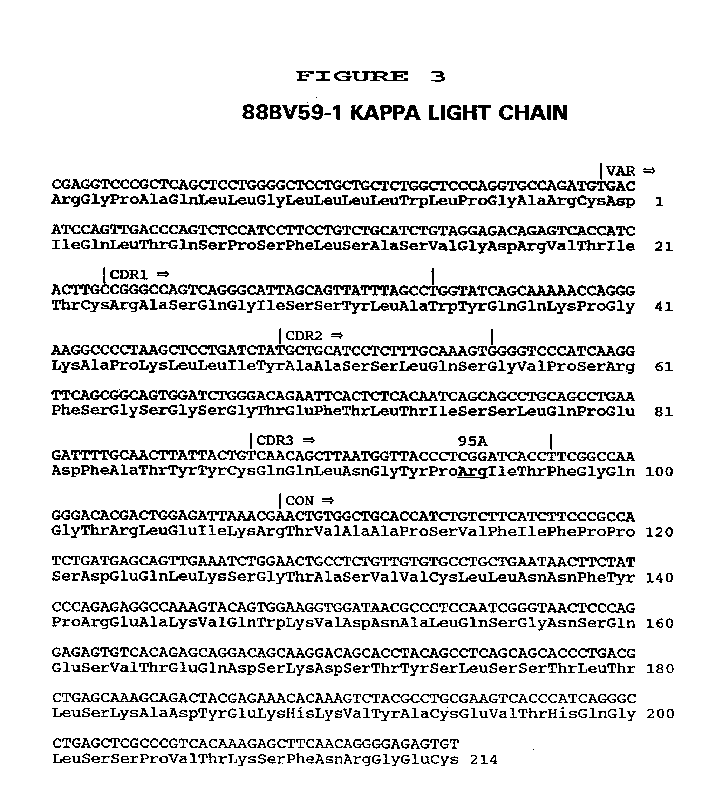

In Figure 2 the 88BV59 kappa light chain sequence is indicated by the positions of the CDRs and the constant region exon. 88BV59 utilizes $V_x$I and $J_x$5. The first $NH_2$ terminal 22 residues were confirmed by amino acid sequencing.

It is of note that a cysteine at amino acid position 59 is present within the 88BV59 $V_H$. This cysteine has been confirmed both from the PCR derived sequence of multiple clones and from the biologically amplified subclone λ phage as well. No other human variable region heavy chains have a cysteine at this position, i.e., Kabat position 59. However, other rare immunoglobulins, i.e., approximately ≤ 1%, have cysteines located within their CDRs as determined either by amino acid or DNA sequencing.

EP 0 546 634 A2

## TABLE 1

## CRITERIA FOR SUCCESSFUL VACCINES FOR
## ACTIVE SPECIFIC IMMUNOTHERAPY

Adjuvant

    (a)   BCG (Phipps, Tice, Connaught); Lyophilized, frozen (dose-dependence $> 10^6$ ($10^7$–$10^8$)

    (b)   C. parvum (Wellcome Labs) (dose-dependence $> 7$ $\mu$g (70 $\mu$g–700$\mu$g)

Tumor Cells

    (a)   Enzymatic dissociation
        (1)   Collagenase type I (1.5–2.0 U/ml HBSS)
        (2)   DNAase (450 D.U./ml HBSS)
        (3)   37°C with stirring
    (b)   Cryopreservation
        (1)   Controlled-rate freezing (-1°C/min) (7.5% DMSO, 5% HSA, HBSS)
        (2)   Viability 80%
    (c)   X-irradiation
        (1)   Rendered non-tumorigenic at 12,000 – 20,000 R.

Components and Administration[1]

    (a)   Ratio of adjuvant to tumor cells – 10:1 – 1:1 (optimum)
    (b)   $10^7$ tumor cells (optimum)
    (c)   2-3 i.d. vaccinations at weekly intervals. Third vaccination contains tumor cells only.

---

[1]    Isoniazid chemoprophylaxis of BCG infection optional.
BCG  – Bacillus Calmette Guerin
HBSS – Hanks' Balanced saline solution
DMSO – Dimethylsulfoxide
HSA  – Human serum albumin
R     – Rads
PBS  – Phosphate buffered saline
EDTA – Ethylenediaminetetraacetic acid

11

TABLE 2

REACTIVITY OF HUMAN MONOCLONAL ANTIBODY 88BV59
Indirect Immunofluorescence with Acetone-filed Tumor Cell Lines[a]

| Cell Line | Tumor Type | Fluorescence Intensity[a] |
|---|---|---|
| Ht-29 | Colon Carcinoma | 3+ |
| SKCO-1[c] | Colon Carcinoma | 3+ |
| LS174 | Colon Carcinoma | 4+ |
| WiDr | Colon Carcinoma | N.T.[e] |
| HCT-8 | Colon Carcinoma | - |
| Bt-20[b] | Breast Carcinoma | 3+ |
| EP[b] | Breast Carcinoma | 2+ |
| MCF-7 | Breast Carcinoma | 4+ |
| SKBR-III | Breast Carcinoma | - |
| CaLu-1[c] | Lung Adenocarcinoma | 4+ |
| A2780 | Ovarian Carcinoma | - |
| Ovcar3[c] | Ovarian Carcinoma | 4+(30%)[d] |
| WI-38 | Normal Fibroblasts | - |

a)   Florescence Intensity: 4+strong, 3+moderate, 2+weak to moderate, 1+ weak, -negative.  Concentration of 88BV59 was 10 $\mu$g/ml.  Staining with a control human IgG at 10 $\mu$g/ml was negative on all cells.
b)   Staining preferentially on cells in mitosis.
c)   Staining shows a filamentous cytoskeletal staining pattern.
d)   Percentage of cells showing the indicated fluorescence intensity was 100% unless otherwise noted.
e)   NT = not tested.

12

TABLE 3

REACTIVITY OF 88BV59 WITH VARIOUS TUMOR TYPES

| Tumor Type | Number of Reactive Tissues | Total Number of Tissues Tested | Percentage |
|---|---|---|---|
| Colon | 74 | 84 | 88 |
| Breast | 22 | 23 | 99 |
| Ovarian | 16 | 20 | 80 |
| Pancreatic | 14 | 17 | 88 |
| Lung | | | |
|    Adenocarcinoma | 6 | 6 | 100 |
|    Squamous cell | 7 | 8 | 88 |
|    Small cell | 0 | 1 | 0 |
| Prostate | 4 | 6 | 67 |
| Melanoma | 0 | 9 | 0 |

TABLE 4

| NAME | SEQUENCE | NUCLEOTIDES (VH=+1) | AMINO ACIDS (VH=+1) |
|---|---|---|---|
| H15-1 | 5'GGAGTTTGGGCTGAGCTG3' | -55→-38 | -19→-13 in $V_H$ leader |
| H15-C | 5'ACCTCCCAGGCTGGACCAC3' | 46→28 | 17→11 in $V_H$ |
| H15-A | 5'GCAGGTACAGCGTGTTCTTG3' | 244→224 | 81→74 in $V_H$ |
| H15-B | 5'GGAAGTAGTCCTTGACCAGG3' | 474→455 | 149→ 142 in $CH_1$ |
| CH1-5' | 5'TCCACCAAGGGCCCATCGGT3' | 379→399 | 115→121 in $CH_1$ |
| H204→208 | 5'CTACACCTGCAACGTGAATC3' | 614→633 | 205→212 in Hinge |

| NAME | SEQUENCE | NUCLEOTIDES (VK= +1) | AMINO ACIDS (VK= +1) |
|---|---|---|---|
| VK 48-43 | 5'TAGATCAGGAGCTTAGGGGC3' | 146→127 | 49→43 |
| VK 10-16 | 5'GTCTGATCTGTAGGAGACAG3' | 33→53 | 11→18 |
| VK 82-88 | 5'GCAACTTATTACTGTCAACAG3' | 250→270 | 84→90 |
| K 170-177 | 5'AGCAAGGACAGCACCTACA3' | 507-526 | 168→175 |

**Claims**

1. Transformed human B-cell 88BV59, ATCC accession number CRL 10624.

2. Monoclonal antibody produced by a cell according to claim 1.

3. Epitope reactive with the monoclonal antibody of claim 2.

4. Monoclonal antibody reactive with the epitope of claim 3.

5. An amino acid sequence comprising the $V_H$ variable region amino acids 1 through 113 in Figure 2 and CDR subunits thereof.

6. The amino acid sequence of claim 5 identified as CDR1 in Figure 2.

7. The amino acid sequence of claim 5 identified as CDR2 in Figure 2.

8. The amino acid sequence of claim 5 identified as CDR 3 in Figure 2.

14

9. An amino acid sequence comprising the $V_x$ chain variable region amino acids 1 through 108 in Figure 3, and CDR subunits thereof.

10. The amino acid sequence of claim 9 identified as CDR1 in Figure 3.

11. The amino acid sequence of claim 9 identified as CDR2 in Figure 3.

12. The amino acid sequence of claim 9 identified as CDR3 in Figure 3.

EP 0 546 634 A2

FIGURE 1

**HEAVY CHAIN**

$375$

LEADER $\longleftarrow V_H \longrightarrow$ $\longleftarrow C_H1 \longrightarrow$

H

-57 100 200 300 400 500 600 669

H15-1 H15-C H15-A $C_{H1}5'$ H15-B H204-208

**LIGHT CHAIN**

LEADER $\longleftarrow V_K \longrightarrow$ $\longleftarrow C_K \longrightarrow$

H

327

100 200 300 400 500 600 648

$V_K 10-16$ $V_K$ $V_K$ $V_K$
48-43 82-88 170-177

16

# FIGURE 2

## 88BV59-1 HEAVY CHAIN

```
                                                            |VAR →
ATGGAGTTTGGGCTGAGCTGGGTTTTCCTTGTTGCTCTTTTAAGAGGTGTCCAGTGTCAG
MetGluPheGlyLeuSerTrpValPheLeuValAlaLeuLeuArgGlyValGlnCysGln    1

GTGCAGCTGGTGGAGTCTGGGGGAGGCGTGGTCCAGCCTGGGAGGTCCCTGAGACTCTCC
ValGlnLeuValGluSerGlyGlyGlyValValGlnProGlyArgSerLeuArgLeuSer   21
                           |CDR1 →               |
TGTGCAGCCTCTGGATTCACCTTCAGTAGCTATGGCATGCACTGGGTCCGCCAGGCTCCA
CysAlaAlaSerGlyPheThrPheSerSerTyrGlyMetHisTrpValArgGlnAlaPro   41
                          |CDR2 →    52a
GGCAAGGGGCTGGATTGGGTGGCAGTTATATCATATGATGGAAGTAATGAATATTGTGCA
GlyLysGlyLeuAspTrpValAlaValIleSerTyrAspGlySerAsnGluTyrCysAla   60
                |
GACTCCGTGAAGGGCCGATTCACCATCTCCAGAGACAATTCCAAGAACACGCTGTACCTG
AspSerValLysGlyArgPheThrIleSerArgAspAsnSerLysAsnThrLeuTyrLeu   80
    82 82a 82b                                 |CDR3 →
CAAATGAACAGCCTGAGAGCTGAGGACACCGCTGTCTATTACTGTGTGAAAGAAGGGTTT
GlnMetAsnSerLeuArgAlaGluAspThrAlaValTyrTyrCysValLysGluGlyPhe   97
      100 100a 100b 100c 100d 100e 100f 100g 100h    |
GGTTCAGTAGTGGTTATTACTCATCTTGCTTTTGATGTCTGGGGCCAAGGGACAATGGTC
GlySerValValValIleThrHisLeuAlaPheAspValTrpGlyGlnGlyThrMetVal  109
      |CH1 →
ACCGTCTCTTCAGCTTCCACCAAGGGCCCATCGGTCTTCCCCCTGGCGCCCTGCTCCAGG
ThrValSerSerAlaSerThrLysGlyProSerValPheProLeuAlaProCysSerArg  129

AGCACCTCTGGGGGCACAGCGGCCCTGGGCTGCCTGGTCAAGGACTACTTCCCCGAACCG
SerThrSerGlyGlyThrAlaAlaLeuGlyCysLeuValLysAspTyrPheProGluPro  151

GTGACGGTGTCGTGGAACTCAGGCGCCCTGACCAGCGGNGTGCACACCTTCCCNGCTGTC
ValThrValSerTrpAsnSerGlyAlaLeuThrSerGlyValHisThrPheProAlaVal  177

CTACAGTCCTCAGGACTCTACTCCCTCAGCAGCGTGGTGACCGTGCCCTCCAGCAGCTTG
LeuGlnSerSerGlyLeuTyrSerLeuSerSerValValThrValProSerSerSerLeu  198

GGCACCCAGACCTACACCTGCAACGTGAATCACAAGCCCAGCAACACCAAGGTGGACAAG
GlyThrGlnThrTyrThrCysAsnValAsnHisLysProSerAsnThrLysValAspLys  221
      |HINGE→                                        END FAB'
AGAGTTGAGCTCAAAACCCCACTTGGTGACACAACTCACACATGCCCACGGTGCCCAGAG
ArgValGluLeuLysThrProLeuGlyAspThrThrHisThrCysProArgCysProGlu  241C

CCCAAATCTTGTGACACACCTCCCCCGTGCCCACGGTGCCCAGAGCCCAAATCTTGTGAC
ProLysSerCysAspThrArgProProCysProArgCysProGluProLysSerCysAsp  241W

ACACCTCCCCCATGCCCACGGTGCCCAGAGCCCAAATCTTGTGACACACCTCCCCCATGC
ThrProProProCysProArgCysProGluProLysSerCysAspThrProProProCys  241QQ

CCACGGTGC
ProArgCys 242
```

In the $V_H$ region, convention Kabat and Wu aa 1-113 are noted
In the $C_H$ region, convention Kabat and Wu aa 114-242 are noted

# FIGURE 3

## 88BV59-1 KAPPA LIGHT CHAIN

```
                                                          |VAR ⇒
CGAGGTCCCGCTCAGCTCCTGGGGCTCCTGCTGCTCTGGCTCCCAGGTGCCAGATGTGAC
ArgGlyProAlaGlnLeuLeuGlyLeuLeuLeuLeuTrpLeuProGlyAlaArgCysAsp    1

ATCCAGTTGACCCAGTCTCCATCCTTCCTGTCTGCATCTGTAGGAGACAGAGTCACCATC
IleGlnLeuThrGlnSerProSerPheLeuSerAlaSerValGlyAspArgValThrIle   21

     |CDR1 ⇒                                  |
ACTTGCCGGGCCAGTCAGGGCATTAGCAGTTATTTAGCCTGGTATCAGCAAAAACCAGGG
ThrCysArgAlaSerGlnGlyIleSerSerTyrLeuAlaTrpTyrGlnGlnLysProGly   41

                     |CDR2 ⇒                     |
AAGGCCCCTAAGCTCCTGATCTATGCTGCATCCTCTTTGCAAAGTGGGGTCCCATCAAGG
LysAlaProLysLeuLeuIleTyrAlaAlaSerSerLeuGlnSerGlyValProSerArg   61

TTCAGCGGCAGTGGATCTGGGACAGAATTCACTCTCACAATCAGCAGCCTGCAGCCTGAA
PheSerGlySerGlySerGlyThrGluPheThrLeuThrIleSerSerLeuGlnProGlu   81

                     |CDR3 ⇒                95A        |
GATTTTGCAACTTATTACTGTCAACAGCTTAATGGTTACCCTCGGATCACCTTCGGCCAA
AspPheAlaThrTyrTyrCysGlnGlnLeuAsnGlyTyrPro**Arg**IleThrPheGlyGln 100

                     |CON ⇒
GGGACACGACTGGAGATTAAACGAACTGTGGCTGCACCATCTGTCTTCATCTTCCCGCCA
GlyThrArgLeuGluIleLysArgThrValAlaAlaProSerValPheIlePheProPro  120

TCTGATGAGCAGTTGAAATCTGGAACTGCCTCTGTTGTGTGCCTGCTGAATAACTTCTAT
SerAspGluGlnLeuLysSerGlyThrAlaSerValValCysLeuLeuAsnAsnPheTyr  140

CCCAGAGAGGCCAAAGTACAGTGGAAGGTGGATAACGCCCTCCAATCGGGTAACTCCCAG
ProArgGluAlaLysValGlnTrpLysValAspAsnAlaLeuGlnSerGlyAsnSerGln  160

GAGAGTGTCACAGAGCAGGACAGCAAGGACAGCACCTACAGCCTCAGCAGCACCCTGACG
GluSerValThrGluGlnAspSerLysAspSerThrTyrSerLeuSerSerThrLeuThr  180

CTGAGCAAAGCAGACTACGAGAAACACAAAGTCTACGCCTGCGAAGTCACCCATCAGGGC
LeuSerLysAlaAspTyrGluLysHisLysValTyrAlaCysGluValThrHisGlnGly  200

CTGAGCTCGCCCGTCACAAAGAGCTTCAACAGGGGAGAGTGT
LeuSerSerProValThrLysSerPheAsnArgGlyGluCys 214
```